# EUROPEAN PATENT APPLICATION

(11) **EP 1 270 010 A2**
(43) Date of publication of application: **02.01.2003**
(21) Application number: 02018944.5
(22) Date of filing: 12.05.1997
(51) Int. Cl.: A61K 38/17

(54) **Use of Macrophage Inflammatory Protein-4 or a fragment thereof for treating adult respiratory distress syndrome**

(30) Priority: 14.05.1996 US 17871 P
(62) Divisional of application: 97303204.8
(71) Applicant: SmithKline Beecham Corporation, Pennsylvania, PA 19406 (US)
(72) Inventor: White, John Richard, c/o SmithKline Beecham Corp., Pennsylvania, PA 19406 (US); Pelus, Louis Martin, c/o SmithKline Beecham Corp., Pennsylvania, PA 19406 (US)
(74) Representative: Crump, Julian Richard John

(57) **Abstract**

The invention relates to the method prevention and treatment of ARDS using Macrophage Inflammatory Protein-4 or biologically active fragment thereof, alone or in conjunction with an anti-infective agent.

## Description

This invention relates to the prevention and treatment of adult respiratory distress syndrome using certain chemokines or biologically active fragments thereof alone or in combination with an anti-infective agent or hematopoietic maturing agent.

Sepsis, as used herein, is broadly defined to mean situations when the invasion of a host by a microbial agent is associated with the clinical manifestations of infection including but not limited to:
(1) temperature >38°C or <36°C; (2) heart rate >90 beats per minute; (3) respiratory rate >20 breaths per minute or PaCO₂ <32 mm Hg; (4) white blood cell count >12,000/cu mm, <4,000/cu mm, or >10% immature (band) forms; (5) organ dysfunction, hypoperfusion, or hypotension. Hypoperfusion and perfusion abnormalities may include, but are not limited to lactic acidosis, oliguria, or an acute alteration in mental states. (Chest 1992; 101: 1644-1566)

Sepsis can occur in hospitalized patients having underlying diseases or conditions that render them susceptible to bloodstream invasion or in burn, trama or surgical patents. In many cases of sepsis, the predominant pathogen is Escherichia coli, followed by other Gram-negative bacteria such as the Klebsiella-Enterobacter-Serratia group and then Pseudomonas. Although comprising a somewhat smaller percentage of infection, Gram-positive microbes such as Staphylococcus and systemic viral and fungal infections are included by the term sepsis as used herein. The genitourinary tract is the most common site of infection, the gastrointestinal tract and respiratory tract being the next most frequent sources of sepsis. Other common foci are wound, burn, and pelvic infections and infected intravenous catheters.
A serious consequence of bacterial sepsis often is septic shock. Septic shock is characterized by inadequate tissue perfusion, leading to insufficient oxygen supply to tissues, hypotension and olgiuria.
Septic shock occurs because bacterial products react with cells and components of the coagulation, complement, fibrinolytic and bradykinin systems to release proteases which injure cells and alter blood flow, especially in the capillaries.
Microorganisms frequently activate the classical complement pathway, and endotoxin activates the alternative pathway. Complement activation, leukotriene generation and the direct effects of bacterial products on neutrophils lead to accumulation of these inflammatory cells in the lungs, release of their proteolytic enzymes and toxic oxygen radicals which damage the pulmonary endothelium and initiate the adult respiratory distress syndrome ("ARDS"). ARDS is a major cause of death in patients with septic shock and is characterized by pulmonary congestion, granulocyte aggregation, haemorrhage and capillary thrombi.
Septic shock is a major cause of death in intensive care units. There are an estimated 200,000 cases per year of septic shock in the United States, and despite advances in technology (i.e., respiratory support) and antibiotic therapy, the mortality rate for septic shock remains in excess of 40%. In fact, mortality for established septic shock has decreased very little since the comprehensive description by Waisbren (Arch. Intern. Med. 88:467-488 (1951)). Although effective antibiotics are available, and there is an increased awareness of the septic shock syndrome, the incidence of septic shock over the last several decades has actually increased. With the appreciation that antimicrobial agents have failed to completely abrogate septic mortality, it is clear that other agents must be developed to be used alone or in conjunction with antimicrobials in order to rectify the deficiencies of current established therapy.

This invention relates to the prevention and treatment of ARDS by administering to an animal, including humans, in need thereof an effective amount of chemokine protein or biologically active fragments thereof.
This invention further relates to the prevention and treatment of ARDS by administering to an animal (including humans) in need thereof an effective amount of chemokine protein or biologically active fragments thereof, either before, in conjunction with or after an anti-infective agent.

It is an object of this invention to use an effective amount of Macrophage Inflammatory Protein-4 or a biologically active fragment thereof, alone or in combination with other anti-infective agents, in the manufacture of a medicament for the treatment of ARDS in an animal, including humans. The chemokine, Macrophage Inflammatory Protein-4, is disclosed in WO95/17092.

The invention further relates to the use of an effective amount of Macrophage Inflammatory Protein-4 or a biologically active fragment thereof, alone or in combination with other anti-infective agents, in the manufacture of a medicament for the prevention of ARDS in an animal, including humans.
Known anti-infective agents include, without limitation, anti-microbial agents routinely used for the treatment of sepsis such as amino-elycosides (such as amikacin, tobramycin, netilmicin, and gentamicin), cephalosporins such as ceftazidime, related beta-lactam agents such as maxalactam, carbopenems such as imipenem, monobactam agents such as aztreonam; ampicillin and broad-spectrum penicillins, (e.g., penicillinase-resistant penicillins, ureidopenicillins or antipseudomonal penicillin or Augmentin) that are active against P. aeruginosa, Enterobacter species, indole-positive Proteus species, and Serratia. Also included within the definition of anti-infective agents are antifungal agents, amphotericin and the like as well as anti-viral agents such as famvir and acyclovir.

The compound is useful in the treatment and prevention of ARDS in humans and other animals such as dairy cattle, horses, calves or poultry.

The use of chemokine protein or biologically active fragments thereof for the prevention and treatment of sepsis has not been reported. It has now been discovered that chemokine protein or biologically active fragments thereof significantly increases the survival of animals challenged with lethal sepsis causing organisms. Treatment with the compound of this invention, alone or in combination with an anti-infective agent prior to contemplated thoracic or abdominal surgery would be useful in reducing the likelihood of post-operative sepsis. It may also be used postoperatively for the treatment of sepsis and ARDS caused by a variety of reasons as outlined previously.

To effectively treat a human or other animal Macrophage Inflammatory Protein-4 or a biologically active fragment thereof may be adminitered by injection in the dose range of about 10 fg/kg to about 100mg/kg/dose, preferably between about 1 and 50mg/kg/dose, or orally in the dose range of about 10 fg/kg to about 100mg/kg body weight per dose, preferably between about 1 and 50mg/kg body weight; if administered by infusion or similar techniques, the dose may be in the range of about 10 fg/kgto about 100mg/kg/dose, preferably between about 1 and 50mg/kg/dose; if administered subcutaneously the dose may be in the range of about 10 fg/kg to about 100 mg/kg/dose, preferably between about 1 and 50mg/kg/dose.

Depending on the patient's condition, the compound of this invention can be administered for prophylactic and/or therapeutic treatments. In therapeutic application, the compound is administered to a patient already suffering from a disease in an amount sufficient to cure or at least partially arrest the disease and its complications. It may be given at any time after surgery, preferably prior to 24 hours after surgery. In prophylactic applications, a composition containing Macrophage Inflammatory Protein-4 or a biologically active fragment thereof, is administered to a patient not already in a disease state to enhance the patient's resistance. It may be given one day or one week prior to surgery, preferably one to two days prior to surgery. It may be administered parenterally or orally.
Single or multiple administrations of the compound can be carried out with dose levels and pattern being selected by the treating physician. In any event, a quantity of the compound of the invention sufficient to effectively treat the patient should be administered.
The compound of this invention, may also be administered in conjunction with a conventional anti-infective as disclosed herein above, such a gentamicin, augmentin or ceftazidime. The particular anti-infective chosen should be one to which the infective organism is susceptible and is selected or modified during therapy as the infecting microrganism is more particularly identified.

Additionally, various adjunctive agents in the treatment of septic shock also may be useful in combination with the components of this invention. They include sympathomimetic amines (vasopressors) such as norepinephrine, epinephrine, isoproterenol, dopamine, and dobutamine; anti-inflammatory agents such as methylprednisolone anti-inflammatory agents such as indomethacin and phenylbutazone; and corticosteroids such as betamethasone, hydrocortisone, methylprednisolone, or dexamethasone; anti-coagulants such as heparin, anti-thrombin III or coumarin type drugs for certain conditions and schedules; diuretics such as furosemide or ethacrynic acid; and antagonist of opiates and beta-endorphins such as naloxone; an antagonist of tumor necrosis factor or of interleukin-1; phenothiazines; anti-histamines; glucagon; α-adrenergic blocking agents, vasodilators; plasma expanders; packed red blood cells; platelets; cryoprecipitates; fresh frozen plasma; bacterial permeability protein; clindamycin; and antibodies to (lipid A), the J5 mutant of E. coli or to endotoxin core glycolipids. Methods for preparing such antibodies are described widely in the literature.
One of the most important aspects in the treatment of the clinical septic shock syndrome is its apparently intractable resistance to the effects of a variety of highly potent antimicrobial agents. Despite the development of newer antimicrobial agents, the overall incidence of clinical sepsis has increased, and mortality remains unacceptably high, often approaching 60% of diagnosed patients. The discovery of the increased survival with the treatment of chemokine protein or biologically active fragments thereof both prophylactically and after infection provides a new and useful therapy of sepsis and ARDS.
The compound of this invention, may also be administered in conjunction with hematopoietic maturation agents, such as G-CSF, Flt3, M-CSF or GM-CSF. These compounds affect the mobilization of the chemokine of the invention and are believed to enhance the anti-sepsis and anti-ARDS efficacy of chemokines.
The biological activity of chemokine protein or biologically active fragments thereof are demonstrated by the following assays:
Rats. Male Fischer 344 rats obtained from Taconic farms weighing 200 to 250 g. are utilized. The rats are housed 2 per cage in standard plastic caging and fed lab chow and water *ad libitum.*

Chemokine protein, or biologically active fragments thereof, is prepared in *E. coli.* The compound is dissolved in DPBS containing 0.5% heat inactivated autologous normal rat serum. The animals are dosed intraperitoneally with chemokine 24 hours and 2 hours before infection. Control animals are dosed with dilution buffer on the same schedule. Starting two hours after infection the rats are treated twice daily with subcutaneous gentamicin.
**E. coli.** A clinical isolate of *E. coli* isolated from sputum is utilized. The organisms are tested for antibiotic sensitivity by the disc-agar diffusion technique and found to be sensitive to gentamicin, ampicillin, cephalothin, chloramphenicol, kanamycin, tetracycline, trimethoprin/sulfamethoxazole and resistant to penicillin G, erythromycin, and vancomycin. The organism is animal passed in mice and subsequently recovered and plated onto MacConkey's agar. The reisolated organisms are grown overnight in brain-heart infusion broth, and then stored frozen at -70°C. The inoculate the fibrin clot, organisms from thawed stocks are inoculated into brainheart infusion broth and incubated overnight on a rotary shaker (120 rpm) an 37°C. The *E. coli* is harvested by centrifugation, washed 3X and finally resuspended in normal saline. The number or organisms is quantified by turbidimentry, and the concentration adjusted with normal saline. All inoculum sizes are based on viable counts determined by scoring colony forming units on MacConkeys agar.
**Fibrin Clot.** The *E. coli* infected fibrin clots are made from a 1% solution of bovine fibrinogen (Type 1-S, Sigma) in sterile saline. The clot is formed by adding sequentially human thrombin (Hanna Pharma.) bacteria, and fibrinogen solution to 24 well plastic plates. Bacterial numbers of 2.0 to 3.0 x 109 are used in inoculate the fibrin clots. The resulting mixture is then incubated at room temperature for 30 minutes before implantation.
**Animal Model.** The rats are anethetized with ketamine/xylazine (40 mg/kg/5 mg/kg) then the abdominal surfaced is shaved and a midline laporatomy performed. Bacterial peritonitis is induced by implanting a fibrin-thrombin clot containing *E. coli* into the abdominal cavity. After implantation the muscle layers are closed with 4-0 silk suture, and the wound closed with surgical staples. The animals are closely observed, any animals obviously moribound are euthanized.
**Gentamicin.** Rats are treated subcutaneously with gentamicin sulfate (Elkins-Sinn, NJ) 5 mg/kg twice a day for five days.
**Statistics.** All continuously variable data are expressed as the percent survival from several pooled studies. The Fisher's Exact test is used to determine the statistical significance of the differences between the survival rates at 14 days. The differences between the groups are considered statistically significant at p<0.05.

### Example 1

### Prophylactically Administered Chemokine.

Chemokine proteins may be prepared using known methods for protein purification or as described in the patent applications listed in Table 1.
The animals are dosed intraperitoneally with chemokine at doses of 10, 100 and 1,000 fg/kg, and 10 and 100 mg/kg 24 hours and 2 hours before infection. Control animals are dosed with dilution buffer on the same schedule. Starting two hours after infection the rats are treated twice daily with subcutaneous gentamicin. On day 0 the rats are implanted with an *E. coli* containing fibrin-thrombin clot. Starting two hours after infection the rats are treated with gentamicin twice daily. The rats prophylactically treated with chemokine at 33 or 100 fg/kg followed by gentamicin treatment demonstrated significantly improved survival rates over the diluent treated control rat receiving gentamicin therapy alone.

### Example 2

### Theraputically Administered chemokine.

On day 0 the rats are implanted with an *E. coli* containing fibrin-thrombin clot. The animals are dosed intraperitoneally with chemokine at doses of 10, 100 and 1,000 fg/kg, and 10 and 100 mg/kg as a single injection 2 hours after infection. Control animals are dosed with dilution buffer on the same schedule. Starting two hours after infection the rats are treated twice daily with subcutaneous gentamicin. The rats theraputically treated with chemokine at 100 or 333 fg/kg followed by gentamicin treatment are assessed for improved survival rates over the diluent treated control rat receiving gentamicin therapy alone.

### Example 3

### Therapeutically Administered chemokine in S. aureus sepsis:

On day 0 the rats are implanted with a *S. aureus* containing fibrin-thrombin clot. The animals are dosed intraperitoneally with chemokine at doses of 10, 100 and 1,000 fg/kg, and 10 and 100 mg/kg as a single injection 2 hours after infection. Control animals are dosed with dilution buffer on the same schedule. Starting two hours after infection the rats are treated twice daily with subcutaneous gentamicin. The rats theraputically treated with chemokine followed by gentamicin treatment are assessed for improved survival rates over the diluent treated control rat receiving gentamicin therapy alone.

## Claims

1. Use of an effective amount of Macrophage Inflammatory Protein-4, or a biologically active fragment thereof, in the manufacture of a medicament for the treatment of ARDS in an animal.

2. Use of Macrophage Inflammatory Protein-4 as claimed in claim 1, wherein the effective amount of Macrophage Inflammatory Protein-4 is about 1 to about 1000mg/kg/dose.

3. Use of Macrophage Inflammatory Protein-4 as claimed in claim 1 or claim 2, wherein said Macrophage Inflammatory Protein-4 or biologically active fragment thereof is administered orally.

4. Use of Macrophage Inflammatory Protein-4 as claimed in any preceding claim, wherein said Macrophage Inflammatory Protein-4 or biologically active fragment is administered subcutaneously.

5. Use as claimed in any preceding claim, where said Macrophage Inflammatory Protein-4 or biologically active fragment thereof is administered 2 hours to 14 hours after surgery.

6. Use of Macrophage Inflammatory Protein-4 as claimed in any preceding claim, wherein said Macrophage Inflammatory Protein-4 or biologically active fragment is administered in conjunction with an effective amount of an anti-infective agent.

7. Use as claimed in claim 6, wherein the anti-infective agent is selected from the group consisting gentamicin, augmentin or ceftazidime.

8. Use of an effective amount of Macrophage Inflammatory Protein-4, or a biologically active fragment thereof, in the manufacture of a medicament for the prevention of ARDS in an animal.

9. Use of Macrophage Inflammatory Protein-4 as claimed in claim 8, wherein the effective amount is about 1 to 100mg/kg/dose.

10. Use as claimed in claims 8 and 9, wherein said Macrophage Inflammatory Protein-4 or biologically active fragment thereof is administered 1 to 2 days prior to surgery.

11. Use as claimed in any of claims 8 to 10, wherein said Macrophage Inflammatory Protein-4 or biologically active fragment thereof is administered in conjunction with an effective amount of an anti-infective agent.

12. Use as claimed in claim 11, wherein anti-infective agent is selected from the group consisting of gentamicin, augmentin or ceftazidime.

13. Use as claimed in any preceding claim, wherein said animal is a human.
